# EUROPEAN PATENT SPECIFICATION

(11) **EP 2 413 828 B1**
(45) Date of publication and mention of the grant of the patent: **31.08.2016**
(21) Application number: 10710526.4
(22) Date of filing: 24.03.2010
(51) Int. Cl.: A61B 18/04, B65D 39/00, A61B 17/42, A61B 18/00

(54) **CERVICAL SEAL**
ZERVIKALDICHTUNG
JOINT CERVICAL

(30) Priority: 02.04.2009 US 166035 P; 23.03.2010 US 729802
(43) Date of publication of application: 08.02.2012
(73) Proprietor: Boston Scientific Scimed, Inc., Natick, MA 01760-1537 (US)
(72) Inventor: NICOLAS, Patrick, Needham Massachusetts 02492 (US); RAO, Doreen, Sudbury Massachusetts 01776 (US); CHU, Michael S.H., Brookline Massachusetts 02446 (US); PETRICCA, John, Ashland Massachusetts 01721 (US)
(74) Representative: Pfenning, Meinig & Partner mbB
(86) International application number: PCT/US2010/028479
(87) International publication number: WO 2010/114751

(56) References cited:
- WO-A1-2007/030002
- WO-A2-2004/047616
- GB-A- 1 111 656
- GB-A- 2 409 201
- US-A1- 2006 047 269
- US-A1- 2008 097 563
- US-A1- 2009 069 796

## Description

### Background

Dysfunctional uterine bleeding may arise from a hormonal imbalance systemic disease or anatomical abnormality such as fibroids, polyps or other growths. A common noninvasive method means of treating such conditions comprises ablation of the lining of the uterus. In hydrothermal ablation ("HTA"), heated saline solution is applied to targeted portions of the endometrial lining via a hysteroscope inserted into the uterus through the cervical canal. However, leakage of the heated fluid from the uterus can damage non-targeted tissue.

A known system for ablating tissue and a corresponding hydrothermal ablation procedure are disclosed in US 2008/0097563 A1. Document WO 2007/030002 A1 suggests for different purposes, in particular diagnostic purposes, to enlarge a uterus using a sealing stopper for sealing the cervix once a liquid has been introduced, so that liquid does not constantly have to be supplied to the uterus.

### Summary of the Invention

The invention relates to a thermal ablation device as defined in the claims. Preferred embodiments are defined in the dependent claims. The present disclosure is directed to a thermal ablation device, comprising an elongated member sized and shaped for insertion into a uterus via a cervical opening, the elongated member defining a first lumen extending therethrough to a first distal opening which, when the apparatus is in an operative position, opens into the uterus and a seal positioned on the elongated member so that, when the elongated member is in the operative position, the seal engages cervical tissue around a circumference of the elongated member and prevent fluids introduced into the uterus via the first lumen from flowing therepast into the cervix, the seal including a plurality of flexible members separated from one another along a length of the elongated member and projecting radially outward from an outer surface of the elongated member.

### Brief Description of Drawings

Fig. 1 shows a known ablation device;
Fig. 2 shows a perspective view of an ablation system according to an embodiment of the invention;
Fig. 3 shows a perspective view of a distal end of the system of Fig. 2;
Fig. 4 shows a side view of the cervical seal of the distal end of the system of Fig. 2;
Fig. 5 shows a side view of the distal end of Fig. 2;
Fig. 6 shows a perspective view of the cervical seal of Fig. 4;
Fig. 7 shows a partially cross-sectional view of a distal end of an ablation system according to a further embodiment of the invention;
Fig. 8 shows a partially cross-sectional view of a distal end of an ablation system according to a still further embodiment of the invention;
Fig. 9 shows a perspective view of a cervical seal for use with an ablation system according to a fourth embodiment of the invention;
Fig. 10 shows a cross-section of a distal end of an ablation system including the cervical seal of Fig. 9;
Fig. 11 shows a perspective view of a cervical seal for use with an ablation system according to a fifth exemplary embodiment of the invention;
Fig. 12A shows a perspective view of a cervical seal for use with an ablation system according to a sixth exemplary embodiment of the invention;
Fig. 12B shows a perspective view of a cervical seal for use with an ablation system according to a seventh exemplary embodiment of the invention;
Fig. 12C shows a perspective view of a cervical seal for use with an ablation system according to a eighth exemplary embodiment of the invention;
Fig. 13A shows a first perspective view of a cervical seal for use with an ablation system according to a ninth exemplary embodiment of the invention;
Fig. 13B shows a second perspective view of the cervical seal of Fig. 13A; and
Fig. 14 shows an expansion device for insertion of a cervical seal according to an exemplary embodiment of the present invention.

### Detailed Description

The present invention may be further understood with reference to the following description and the related appended drawings, wherein like elements are provided with the same reference numerals. The present invention describes a sealing device for preventing the leakage of fluids from the uterus when performing hydrothermal ablation. An exemplary sealing device of the present invention is directed for use with standard HTA trans-cervical sheaths. However, it is noted that the present invention may be employed to enhance the sealing for other ablation device inserted into hollow organs via openings which naturally seal around the devices inserted therethrough.

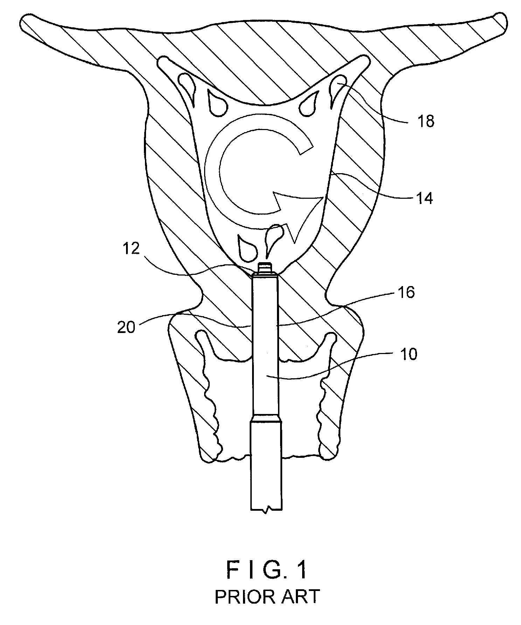

Fig. 1 depicts a known ablation device 10 deployed in an active configuration with a distal tip 12 thereof opening into a uterus 14 of a living body. The ablation device 10 is includes an elongated tubular member 16 with a substantially smooth outer portion sized to fit within the cervical canal 20. A maximum diameter of the ablation device 10 is maintained less than a predetermined maximum diameter to prevent undue trauma to the cervical canal 20. To sealingly engage the cervical walls of most patients without overly stretching the tissue thereof, an outer diameter of the ablation device 10 may, for example, be approximately 7.62 mm. If the outer diameter of the ablation device 10 is insufficient to sealingly engage the walls of the cervical canal 20, the heated fluid 18 may leak out of the uterus 14 and damage non-targeted tissue while decreasing the efficacy of the ablation procedure. A proximal portion of the ablation device 10 further comprises an increased diameter portion which prevents, for example, an insertion of the ablation device 10 into the cervical canal 20 beyond the length of the elongated tubular member 16.

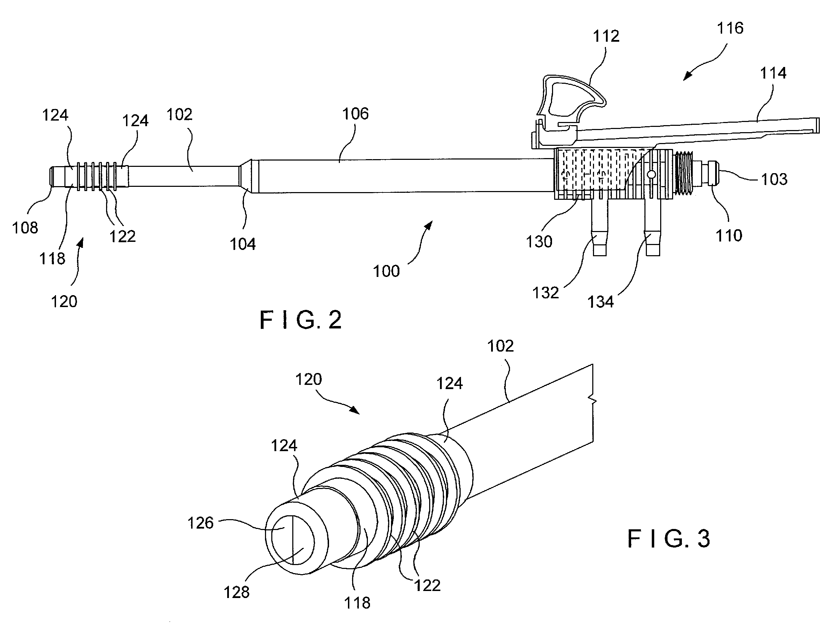

As shown in Figs. 2 - 6, an introducer 100 for an ablation system according to an exemplary embodiment of the invention comprises an elongated body 106 extending from a proximal end 103 which remains outside the body at all times to a distal end 108 which, when in an operative position opens into the uterus 14 via the cervical canal 20 in the same manner as the distal tip 12 of the device 10. In use, as would be understood by those skilled in the art, the introducer 100 is coupled to a known thermal ablation system including a supply of heated fluid and a system for withdrawing the fluid from the body.

Generally, the thermal ablation system may include a console receiving fluid from an intravenous (IV) bag (not shown) supplying ablation fluid thereto at a desired pressure. The IV bag may contain fluid such as saline that will be heated and circulated through the uterus to ablate the endometrial lining. The fluid may be filtered and recirculated or removed from the system after being withdrawn from the body as would be understood by those skilled in the art. During the ablation procedure, an operator (e.g., physician, nurse, etc.) may be required to substitute the IV bag for IV bags with other fluids depending on stage of the ablation procedure. For example, prior to the procedure an anesthetic fluid may be circulated through the uterus to numb the endometrium. Additionally, after the endometrial lining has been ablated, an analgesic and/or infection preventative solution may be circulated through the uterus. The console comprises a housing encasing electronic circuitry and providing a user interface for displaying content (e.g., instructions, procedural data, warnings, etc.) and receiving user input. The user interface may comprise a display screen (e.g., LCD) and a keypad for submitting input to the console. The console effectively controls the flow of fluid into and out of the introducer 100.

As shown in Fig. 2, an introducer 100 according to an exemplary embodiment of the present invention is coupled to a fluid source (not shown) and a fluid reservoir (not shown) via a delivery inlet 134 and a return outlet 132 to deliver fluid to the uterus 14 and to return the fluid from the uterus 14 to the fluid reservoir (not shown). The delivery inlet 134 and the return outlet 132 are respectively coupled to a delivery lumen 126 and a return lumen 128 both extending longitudinally through the introducer 100. The delivery lumen 126 and the return lumen 128 terminate at a distal end 108 of the sheath 102 which, when the introducer 100 is in an operative position, is located within the uterus. The delivery lumen 126 and the return lumen 128 are formed of substantially equal sizes to permit an even circulation of ablation fluid through the uterus. Although the delivery lumen 126 and the return lumen 128 as concentric tubes, it is submitted that lumens of any suitable shape may be employed such as, for example, circular lumens, elliptical lumens, etc. The introducer 100 may optionally include a vision system to allow visualization of the operative area. Those of skill in the art will understand that the vision system may be substantially similar to the systems in conventional endoscopes (e.g., fiber optic or CCD-based systems). Alternatively, users may rely on the vision system of an endoscope or other instrument inserted through the introducer 100, as those skilled in the art will understand.

The introducer 100 includes a handle 130 coupled to the sheath 102 and comprising a scope connector 110 for receiving a visualization device, such as an endoscope. The handle 130 facilitates holding and manipulation of the introducer 100 with a single hand while the operator uses his free hand to interface with a console (not shown) controlling the visualization device, manipulate the patient's anatomy, etc. The scope connector 110 according to this embodiment is disposed on a proximal end of the introducer 100 and provides an attachment point for the visualization device (e.g., a hysteroscope, an endoscope) so that the visualization device may be passed distally through a visualization lumen in the introducer 100 and extended out of the distal end 108. Thus, the operator may visually monitor insertion of the introducer 100 into the uterus.

The visualization device is inserted into the proximal end of the introducer 100 through the scope connector 110 and locked thereto using a means known in the art. Those of skill in the art will understand that the scope connector 110 may be implemented as any mechanism which allows the length of the introducer 100 to be adjusted to and maintained at a new length. For example, a scope adapter (not shown) may be provided as a projection on an outer surface of a distal portion of the scope connector 110 received in and movable between one of a number of locking apertures (not shown) formed on the handle 130. Partial barriers may be formed between each of the locking apertures (not shown) to retain the scope adapter (not shown) in a selected one of the locking apertures (not shown) maintaining a selected length of a portion of the scope connector 110 projecting from the proximal end of the introducer 100. In another embodiment, a rack (not shown) may be formed on the distal portion of the scope connector 110 mating with a gear in the handle 130 so that rotation of the gear extends and withdraws the scope connector 110 relative to the handle 130. A ratchet may be provided to maintain the gear in a fixed position relative to the rack, thereby maintaining the desired position of the scope connector 110 relative to the handle 130.

The introducer 100 of the present invention may also comprise a tenaculum stabilizer 116 on the handle 130. During the ablation procedure, a tenaculum (not shown) may be placed around the cervical canal and coupled to the ablation device 100 to ensure that the ablation device 100 remains at a desired position and depth within the uterus and is not inadvertently withdrawn therefrom. That is, the tenaculum stabilizer 116 ensures that the distal end 108 of the sheath 102 is not withdrawn proximally from the uterus during the procedure to prevent non-targeted tissue from being exposed to the ablation fluid. When the tenaculum is coupled to the tenaculum stabilizer 116, the operator selects a tension to be applied between the tenaculum and the ablation device 100 by moving the fin along the rail 114. A positioning mechanism (e.g., ratchet, latch, clip, etc.) may be used to maintain a position of a fin 112 relative to the rail 114, as would be understood by those skilled in the art.

The introducer 100 further comprises a cervical seal 120 located at a distal end thereof. In contrast to presently available cervical seals, the cervical seal 120 does not require selective actuation in order to engage with the walls of the cervical canal, as will be described in greater detail below. The cervical seal is formed as a flexible mechanical barrier extending from an outer diameter of an introducer 100 to seal a distal portion of the cervical canal 20 to prevent fluid transfer between the uterus and the proximal end of the cervical canal 20. As will be described in greater detail below, the cervical seal 120 comprises a plurality of flexible ribs 122 extending radially outward from the introducer 100 to enhance the engagement between the tissue of the cervical canal 20 and the device 100.

When the introducer 100 is in a desired position with a distal end 108 thereof within the uterus 14, the cervical seal 120 is seated within a distal portion of the cervical canal 20. The introducer 100 comprises a proximal elongated body 106 with a tapered portion 104 at a distal end thereof leading to a sheath 102 adapted to be received within the cervical canal 20. As indicated above, when in an operative position, a distal end 108 of the sheath 102 opens into the uterus 14. An outer diameter of the sheath 102 is preferably smaller than an outer diameter of the elongated body 106 and, in a preferred embodiment, is approximately 8 to 8.5mm. The sheath 102 is formed of a polycarbonate material sufficiently rigid to allow the introducer 100 to be inserted through the cervical canal 20 into the uterus 14 as would be understood by those skilled in the art. Diameters of the delivery lumen 126 and the return lumen 128 extending through the sheath 102 may be maximized to or decreased to affect a flow of heated ablation fluid therethrough. The delivery lumen 126 and the return lumen 128 extend through the length of the introducer 100 and are open at a proximal portion comprising a handle 130 to a delivery inlet 134 and a return outlet 132 and, at a distal end, into the uterus. In use, a heated fluid source is connected to the delivery inlet 134 to pump fluid into a directed body cavity via the delivery lumen 126. After a predetermined duration (e.g., approximately 10 minutes), the heated fluid is removed from the body cavity via the return lumen 128 and out through the return outlet 132, which may be connected to a reservoir for receiving the heated ablation fluid.

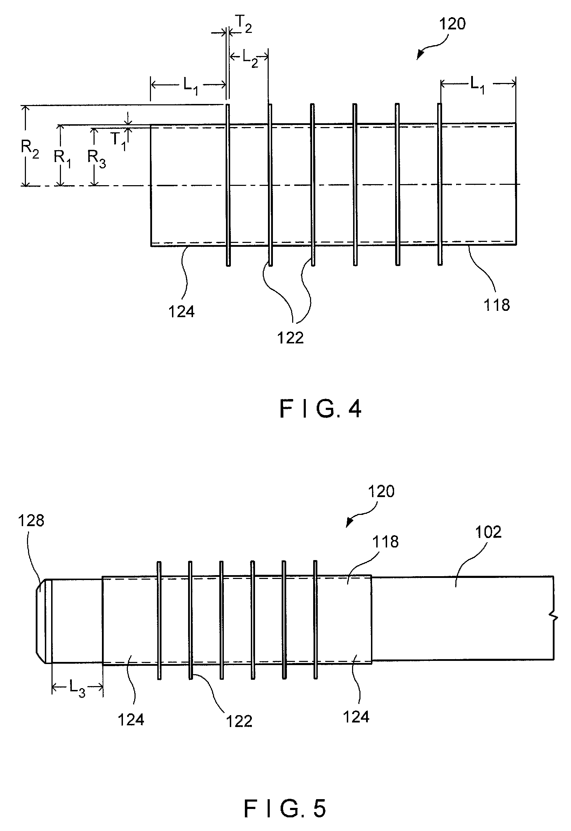

The cervical seal 120 is separated from a distal end 108 of the sheath 102 by a length L₃ indicative of a preferred depth by which the introducer 100 is to abut into the uterus. The length L₃ was chosen to be approximately 5mm to provide an unobstructed and clear translucent space through the base (tube) to cap for the UV bonding process. However, smaller lengths may be used. L₃ may be shortened for other processes where UV bonding is not used. The diameter at L₃ is a smaller (lead in diameter) to allow the tip of the introducer to be position into the canal before the slightly larger diameter of the seal is introduced. The distal end 108 is provided with a rounded edge which prevents trauma to a patient. The cervical seal 120 comprises a base 118 formed over a length of the sheath 102. The base 118 is formed of a silicone material with a thickness T₁ of approximately 0.1778 mm - 0.2032 mm and an outer radius R₁ of approximately 3.886 mm. The base 118 may be formed of silicone or of the same material as the ribs 122 or, as would be understood by those of skill in the art, of any materials having sufficient flexibility at the ablation temperature and which is suitable for sterilization. Silicone rubber or a rubber like material may be desired for their properties such as silicone's resilience to our operating temperature of 90 degrees C and its ability to recover to its normal (molded) state. Alternatively, acrylonitrile butadiene styrene ("ABS"), polyether block amide ("PEBA") may be used as the material for the polycarbonate tube and the base mentioned below in this application. The material needs to be clear if UV bonding is to be used. In a preferred embodiment, an inner diameter of the base 118 and an outer diameter of the polycarbonate sheath 102, which contact one another in a bonded configuration as shown clearly in Fig. 3, can be plasma treated for approximately one minute, as those skilled in the art will understand, to form a bond therebetween. As would be understood by those skilled in the art, an open air ionized gas plasma treatment may be used. The activation energy for the plasma treatment may comprise long or short radio waves or may comprise microwaves, as also known to those skilled in the art. The plasma treatment prepares the contacting surfaces of the sheath 102 and the base 118 for bonding by raising the surface energy of each of the contacting surfaces, thus ensuring a sufficient bond therebetween. Once plasma treatment is complete, a small amount of isopropyl alcohol ("IPA") is applied to the base 118 to allow the base 118 to slide over the plasma treated portion of the sheath 102. The sheath 102 may include markings aiding in positioning the base 118 thereon or the base 118 may be located on the sheath 102 by fixtures (e.g., by placement adjacent to a molded end connector of the sheath 102 as would be understood by those skilled in the art. An adhesive known in the art is then applied to the contacting surfaces of cuffs 124 located on ends of the base 118. The adhesive, such as, for example, Dymax 1-20779, is reactive to ultraviolet light and, once the base 118 of the cervical seal 120 has been properly positioned, is cured for approximately thirty seconds at 1836 mW/cm² power. Also a chamfer or radius is formed at the edge of a length L₁ of approximately 5.08 mm, as shown in Fig. 5. The transition radius which may, for example, be formed as a feathered out bead of bonding glue outside the end of the cuffs of the seal 118 provides a smoother/radius transition from the polycarbonate tube onto the silicone base 118. Alternatively, the base 118 can be secured to the sheath 102 via another means known in the art such as mechanical means (e.g., swaging, tying and sleeving, etc.). The base 118 is preformed with a plurality of circumferential ribs 122 located at equidistant intervals thereover. It is noted however, that any spacing interval may be provided between adjacent ribs 122. It is preferred that the spacing between the ribs 122 is large enough that adjacent ribs 122 do not overlap one another during introduction to minimize the collapsed profile. The ribs 122, which are formed of the same substantially flexible material as the base 118, comprise a thickness T₂ of approximately 0.2032 mm. Each rib extends radially distally of the sheath 102 by approximately to an outer radius R₂ of approximately 5.499 mm, greater than the ribs free radius R₃ of approximately 5.296 mm. The embodiments of Figs. 2 - depict six ribs 122 formed on the base 118, each of the ribs 122 spaced from an adjacent rib 122 by a length L₂ of approximately 2.667 mm. It is noted, however, that any plurality of ribs 122 may be employed without deviating from the spirit and scope of the present invention. In one exemplary embodiment 16 ribs 122 may be employed to allow the entire canal to be in contact with the seal. The 16 ribs 122 may be formed longer than the canal to provide a seal during the hysteroscopy phase where the sheath is moved and manipulated for viewing and to accommodate inadvertent movement during the ablation phrase. Using 16 ribs 122 provides a longer seal allowing the device to be positioned deep within the uterus, at user discretion, while maintaining the desired seal. The ribs 122, which are formed as a unitary element with the base 118, are permitted to deflect proximally or distally with respect to a longitudinal axis extending through the sheath 102. In this manner, the cervical seal 102 is movable between an initial diameter of approximately 11 mm. and a compressed diameter of approximately 8.42 mm. when a pressure is applied to the ribs 122 by the wall of the cervical canal. The compressed configuration enables the cervical seal 102 to frictionally engage the wall of the cervical canal, which, as noted above, is dilated to a nominal 8 mm. Of course, those skilled in the art will understand that some cervical canals will exceed 8mm in diameter even without dilation. The above embodiments of the seal may be overmolded onto the base 118 to avoid the gluing process.

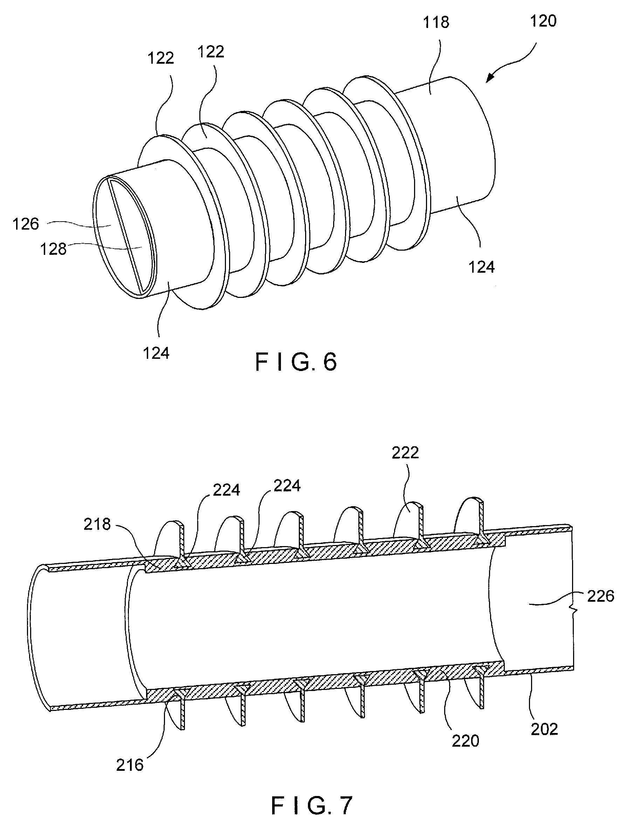

In an alternate embodiment of the present invention, as shown in the partial cross-sectional view of Fig. 7, a cervical seal 220 is formed with substantially the same features of the cervical seal 120 of Figs. 2 - 6. The cervical seal 220, which is formed as a cylindrical element is provided with a plurality of ribs 222 disposed along a plurality of lengths thereof. The ribs 222 are formed as rings encircling a base 218 of the cervical seal 220. Ends 224 of the ribs 222 are formed as protrusions along an inner diameter of the ribs 222. In the exemplary embodiment shown, the ends 224 are formed as triangular protrusions comprising a width greater than the width of the ribs 222 and housed in respectively shaped grooves 216 formed in the base 218. However, it is respectfully submitted that any suitable shape may be employed for the ends 224 For example, the ends 224 may be formed as spheres, rectangular prisms, etc. where the width of the end is at least greater than that of the ribs 222. By molding each of the ribs 222 into the base 218, the rigidity of the cervical seal 220 is strengthened while also bypassing the need for any additional bonding between the base 118 and the ribs 222.

A sheath 202 is machined over the cervical seal 220 via a means known in the art. For example, an outer wall of the sheath 202 in this area may be made thicker to accommodate machining triangular anchor slots thereinto. The sheath 202 is molded with a substantial friction fit to prevent leakage of any fluid therepast while still preventing a substantial radial constriction of the cervical seal 220. The machining process bypasses the need for any additional bonding means between the base 118 and the cervical seal 220. The sheath 202, which is formed of the same material as the cervical seal 220 defines a dual- lumen 226 extending therethrough from a proximal end (not shown) which is open to a heated ablation fluid source to a distal end (not shown) which is open to a uterus in an operative configuration, as described above with respect to Figs. 2 - 6. Specifically, the dual-lumen 226 may comprise both a delivery lumen and a return lumen extending therethrough. Heated ablation fluid flowing through the dual-lumen 226 is substantially undeterred by the cervical seal 220 abutting thereinto due to a relatively negligible thickness thereof, as also disclosed with respect to Figs. 2 - 6.

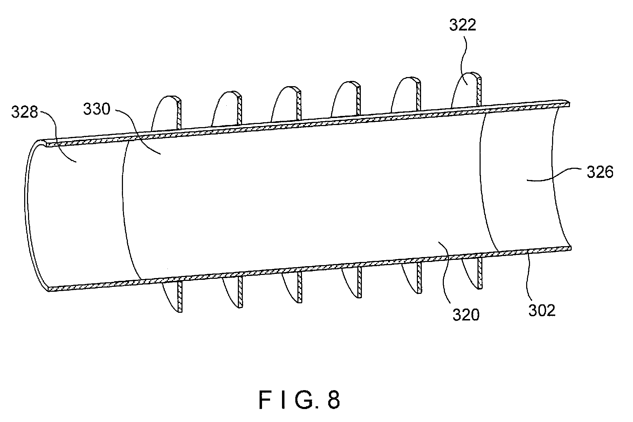

In a further embodiment, as shown in Fig. 8, a cervical seal 330 may be formed substantially similarly to the seal 220 of Fig. 5 except that the seal 330 is overmolded directly to the outer tube as described below to lie flush against an inner wall 328 of a dual-lumen 326 extending through a sheath 302. The sheath 302 is formed as an elongated hollow structure with a recess 316 formed along a length of a distal portion thereof, the recess 316 adapted to receive the cervical seal 320 therein. The cervical seal 320 is formed substantially similarly as shown in Figs. 2 - 6, a base 318 thereof being formed as a unitary element with ribs 322. The sheath 302 is formed over the cervical seal 320 via an overmolding process known in the art. Once formed, an inner wall 330 of the cervical seal 320 lies flush against the inner wall 328 so that any fluid flow through the dual-lumen 326 is not obstructed.

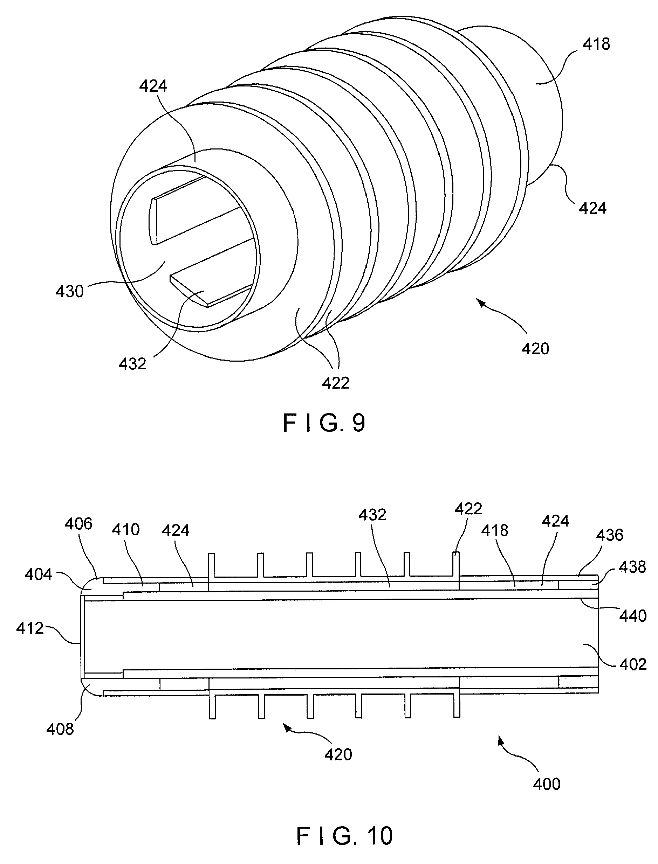

Figs. 9 - 10 show yet another alternate embodiment of the present invention wherein a cervical seal 420 is formed by an overmolding process. Specifically, a plurality of ribs 422 are overmolded onto a base 418. An inner wall 430 of the cervical seal is provided with a plurality of longitudinal members 432 protruding into the base 418 running parallel to a longitudinal axis of the cervical seal 420. The base 418 has recesses in which the members 432 are formed with the members 432 locking the ribs 422 in place relative to the base 418 and is the connection between the ribs 422 during the molding process. Alternatively, the seal 420 may be overmolded on the base 418 obviating the need for the members 432. After the cervical seal 420 of Fig. 9 has been formed, a subsequent overmolding process is performed to join the cervical seal 420 with a sheath 402. The sheath 402, which may be formed with a smaller diameter than disclosed with respect to Figs. 2 - 6, is received within the cervical seal 420 and bonded thereto. UV bonding is then performed to fit an outer sheath 436 over the subassembly, the outer sheath 436 defining a hollow portion 438 therein as well as a dual-lumen 440 extending longitudinally though a central axis thereof. Once the UV bonding process is complete, a distal end 408 of ablation device 400 is fitted with a stopper 404 with a substantial friction fit and UV bonded to seal the hollow portion 438 of the outer sheath 436. The stopper 404 is formed with a rounded distal end 406 in order to minimize any sharp edges on the ablation device which may cause trauma when inserted into a patient and a tapered leg 410 adapted to be received in the hollow portion 436. Once inserted, a proximal end of the tapered leg 410 engages a distal end of the base 418. The rounded edges of the stopper 404 converge at a central face 412 comprising a ring defining a distal opening of the ablation device. All the components in this embodiment may be designed to be inter-fitting and UV bonded to one another. UV bonding works best with transparent materials through which the UV light can pass. The seal 418 may then be over molded (e.g., in a single step). This design was to minimize the base of the seal to equal the diameter of the outer diameter of the sheath 436. The rigid surface of the base 418 allows it to be inserted into the ID of the sheath 436 for UV bonding.

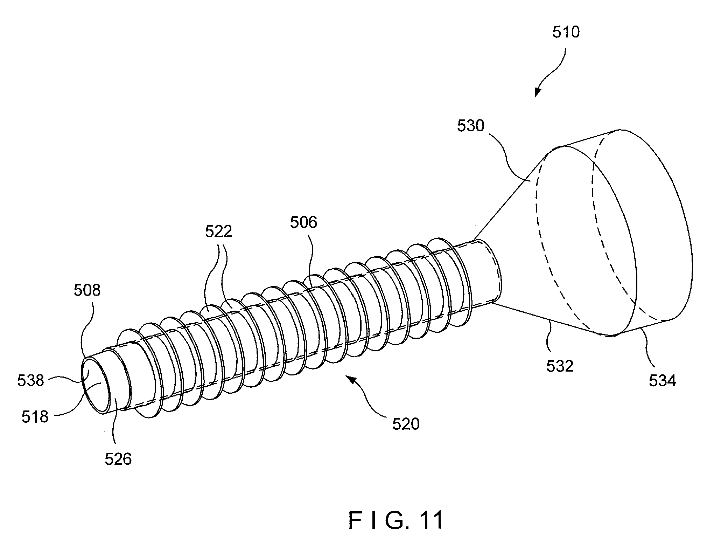

Fig. 11 depicts another embodiment of the present invention, comprising a cervical seal 520 formed on an accessory sheath 510 within which the known ablation device of Fig 1 may be employed. Specifically, the accessory sheath of Fig. 11 may be received over a known ablation device adapted to be inserted into a cervical canal to perform a thermal ablation procedure. The accessory sheath 510 comprises the cervical seal 520 extending from an elongated body 506 formed as a hollow tube. The elongated body 506 extends from a distal end 508 comprising an opening 518 to a proximal end comprising an increased diameter connector 530. The connector 530 follows a taper 532 increasing in diameter in a proximal direction away from the elongated body 506 to a substantially cylindrical proximal portion 534. An inner diameter of the proximal portion 534 is sized to be slightly greater than an outer diameter of an increased diameter portion of an ablation device such as, for example, the increased diameter portion 22 of the ablation device 10 of Fig. 1. In this manner, the connector 530 may be mated with the ablation device to form a secure fluid-tight seal.

A plurality of flexible ribs 522 are distributed along a portion of the length of the elongated body 506. The ribs 522 extend radially outward from the body 506 to engage tissue of a cervical canal, as described in greater detail earlier. Furthermore, a diameter and number of the ribs 522 may be adjusted to conform to the target portion of anatomy within which the device is to reside (e.g., the uterine cervix). Thus, dimensions of the device according to this embodiment may be similar to those disclosed above in regard to the cervical seal 120 of Figs. 2 - 6. The ribs 522, elongated body 506 and the connector 530 which together comprise the accessory sheath 510, may be formed of a substantially flexible silicon material via an overmolding process, as those skilled in the art will understand. To provide the rigidity needed to slide the cervical seal 520 onto a thermal ablation device, the accessory sheath 510 may be overmolded onto a polyimide tube 526. In one exemplary embodiment, the polyimide tube comprises a diameter of approximately 0.0254 mm. An elongated introducer of a thermal ablation device such as, for example, the elongated tubular member 16 of ablation device 10, may then be inserted through the substantially rigid polyimide tube 526 and though a distal opening 538 thereof. A diameter of the polyimide tube 526 may be chosen to be slightly smaller than an outer diameter of the elongated tubular member 16 of the thermal ablation device 10 to ensure that a sufficient frictional seal is formed therebetween.

The device of Fig. 11 may be employed with a method substantially similar to the method discussed with respect to Figs. 2 - 6. Additionally, since the accessory sheath 510 is selectively engageable with the thermal ablation device, a user of the device may decide when employment thereof becomes necessary. For example, the accessory sheath 510 may only be placed over the ablation device 10 when necessary (e.g., when a cervix is dilated to a diameter greater than a nominal 8 mm. diameter, if leakage occurs, etc.).

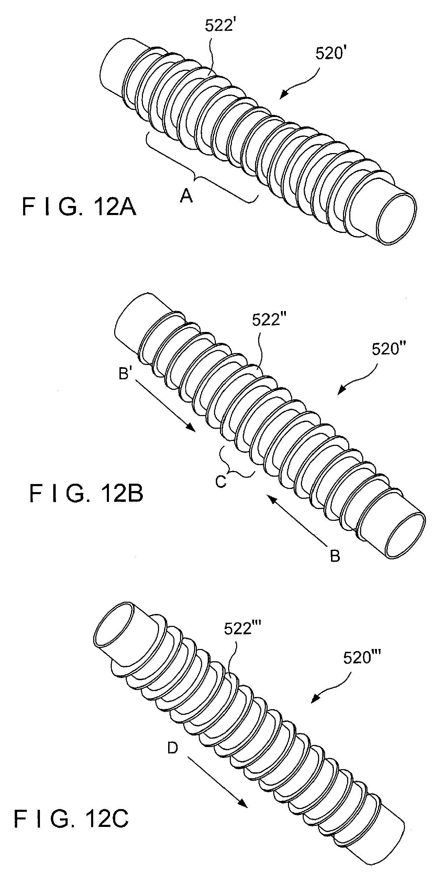

It is noted that although the accessory sheath 510 has been described with respect to thermal ablation procedure, the accessory sheath 510 and variations thereof may be employed in any other medical procedure requiring access to a uterus such as, for example, a biopsy, a tumor resection or a uterus flush, as those skilled in the art will understand. Furthermore, a plurality of modifications may be made to the cervical seal 520. As shown in Figs. 12A - 12C, ribs of the cervical seal 520 may assume any configuration and will not necessarily be circular. In one embodiment, as shown in Fig. 12A, the ribs 522' of a cervical seal 520' are formed in a dumbbell configuration with the ribs 522' assuming a repeating pattern A and tapering down in size from a greater diameter to a smaller diameter. The ribs 522' may fluctuate only from a predetermined maximum diameter to a predetermined minimum outer diameter. Employing the dumbbell shape of Fig. 12A may facilitate insertion of the cervical seal 520' into a cervical canal while ensuring a sufficient frictional fit therewithin. In another embodiment, as shown in Fig. 12B, ribs 522"' may comprise a minimum diameter at proximal and distal ends of the cervical seal 520" tapering to a to a maximum outer diameter toward a center C of the cervical seal 520". Specifically, the ribs 522"' located proximally of the center C may taper in the direction B and ribs 522"' located distally of the center C may taper in the direction B'. Furthermore, as shown in Fig. 12B, the ribs 522" may be profiled into ovals or alternatively, any other shape to provide a better seal against surrounding tissue of the cervical canal. In another embodiment, as shown in Fig. 12C, ribs 522"' may taper down in a direction D from a first end of a cervical seal 520'" to a second end.

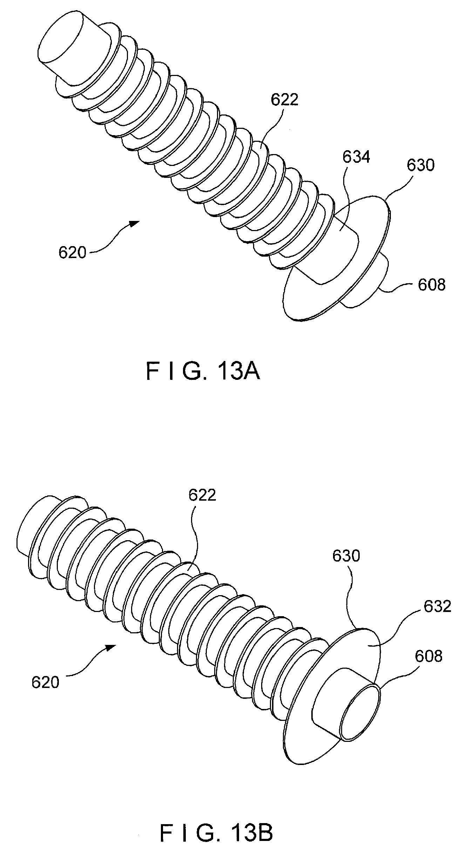

Figs. 13A and 13B depict another alternate embodiment of the present invention wherein a distal portion of a cervical seal 620 is provided with a flap 630 located adjacent a distal end 608. The flap 630 is formed in substantially the same manner as ribs 622 and comprises substantially similar properties with the exception of a diameter thereof which is selected to be greater than a diameter of an orifice of the cervical canal into the uterus, as those skilled in the art will understand. The flap 630 may be formed of the same substantially flexible silicon material as ribs 622. The flap 630 is introduced through the cervical canal and inserted into the uterus so that a proximal face 632 thereof abuts a wall of the uterus adjacent the orifice of the cervical canal. Thus, the flap 630 serves as an additional fluid sealing between the uterus and the cervical canal. Furthermore, a space 634 is provided between the flap 630 and a distalmost one of the ribs 622 to permit the flap 630 to deflect proximally during insertion through the cervical canal without overlapping over the distalmost one of the ribs 622. Those skilled in the art will understand that avoiding this overlapping prevents the profile of the cervical seal 620 from being unduly increased during insertion and reduces patient trauma.

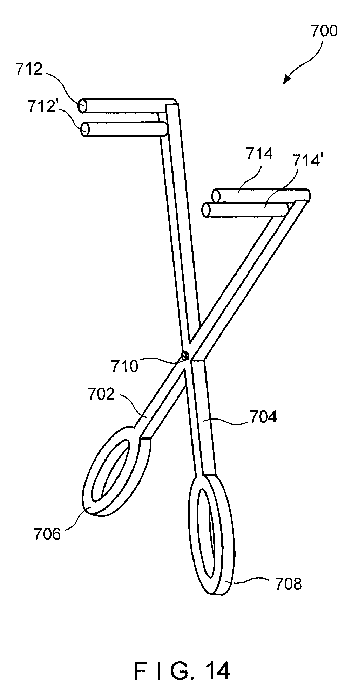

Fig. 14 depicts an exemplary expansion device 700 that may be used to place the accessory sheath 510 of Fig. 11 onto a thermal ablation device. The expansion device 700 is formed as a substantially scissor like device with two arms 702, 704 comprising finger-gripping means 706, 708 at proximal ends thereof. The arms 702, 704 are pivotally attached to one another by a hinge 710 separated from respective finger-gripping means 706 by a predetermined distance. A distal end of each of the arms 702, 704 comprises a pair of protrusions 712, 712' and 714, 714', respectively, extending substantially perpendicularly from the arms 702,704. The protrusions 712, 712', 714, 714' are formed with smooth outer walls and may comprise a substantially cylindrical shape. The protrusion 712 is spaced from the protrusion 712' and the protrusion 714 is spaced from the protrusion 714' other a portion of the length of the respective one of the arms 712, 714.

According to an exemplary method the protrusions 712, 712', 714, 714' of the expansion device 700 are inserted into an inner diameter of the accessory sheath 510. The arms 702, 704 are then manually actuated via the finger-gripping means 706, 708 to separate the respective distal ends from one another. The expansion causes the accessory sheath 510 to expand to a slightly greater diameter, thus allowing the elongated tubular member 16 of the thermal ablation device 10 to be inserted with minimal frictional resistance. Those skilled in the art will understand that the employment of multiple protrusions 712, 712', 714, 714' on the arms 702, 704 decreases a pressure applied to an adjacent portion of a wall of the accessory sheath 510. Specifically, the increase in abutting surface area between the protrusions and the wall of the accessory sheath 510 subjects a lesser expanding pressure on the wall of the accessory sheath 510. Once the accessory sheath 510 has been properly positioned over the elongated tubular member 16, the expansion device 700 is removed and the accessory sheath 510 recovers to its original diameter to assume a compressed configuration against the elongated tubular member 16.

It is noted that the embodiments shown are shown for illustrative and descriptive purposes only and are not intended to describe the bounds of the present invention which is to be limited only by the scope of the claims appended hereto. There are many modifications of the present invention which will be apparent to those skilled in the art without departing from the teaching of the present invention. For example, various modifications may be made to the material, size, shape and number of ribs employed in any of the examples discussed herein.

## Claims

1. A thermal ablation device comprising:
an elongated member (102) sized and shaped for insertion into a uterus via a cervical opening, the elongated member (102) defining a first lumen (126) extending therethrough to a first distal opening which, when the device is in an operative position, is configured to open into the uterus; and which is configured to be connectable at a proximal portion to a heated fluid source; and a seal (120) positioned on the elongated member (102) so that, when the elongated member is in the operative position, the seal (120) engages cervical tissue around a circumference of the elongated member and prevent fluids introduced into the uterus via the first lumen (126) from flowing therepast into the cervix, the seal (120) including a plurality of flexible members (122) separated from one another along a length of the elongated member (102) and projecting radially outward from an outer surface of the elongated member (102), wherein the flexible members (122) are substantially disc-shaped and are deflectable proximally and distally along the elongated member (102) when compressed by contact with surrounding tissue and wherein a spacing between the flexible members (122) prevents one flexible member (122) from overlapping an adjacent flexible member (122), wherein the device further comprises a second lumen (128) extending proximally through the elongated member (102) from a second distal opening which, when the apparatus is in the operative position, is configured to open into the uterus, the second lumen (128) being operative to withdraw fluid from the uterus.

2. The device of claim 1, wherein a diameter of the flexible members (122) changes along a length of the seal (120), the flexible members (122) following a decreasing taper from a first point to a second point along a length of the seal (120).

3. The device of claim 1, wherein the seal (120) is formed of one of a silicone elongate cylindrical fitting (118) bonded to the elongated member (102) and a silicone elongated cylindrical fitting (118) molded into the elongated member (102), the seal (120) further comprising an increased diameter fitting on a proximal end thereof, the increased diameter fitting sized and shaped to slide over the elongated member (102) with a friction fit.

4. The device of claim 3, further comprising grooves (216) molded into the elongated cylindrical fitting (218) adjacent to the seal (120), each of the grooves (216) being sized to receive a corresponding one of the flexible members (222) when compressed thereagainst during insertion through the cervix.

5. The device of claim 1, where each of the flexible members (122) is approximately 0.2 mm thick and has an outer diameter of approximately 11 mm.

6. The device of claim 1, further comprising a flexible flap (630) projecting radially outward from an outer surface of the elongated member (102) distally of the flexible members by a distance greater than a radial projection distance of the flexible members (622), a diameter of the flap (630) being selected to be greater than a diameter of an opening of the cervical canal into the uterus.

7. A thermal ablation system comprising the device of claim 1, the thermal ablation system further comprising a fluid handling unit fluidly coupled to a proximal end of the first lumen (126), the fluid handling unit (134) supplying heated fluid to the first lumen (126), wherein the seal (120) is made of silicone and positioned on a distal portion of the elongated member (102).

8. The system of claim 7, further comprising an introducer (100) coupled to a proximal end of the elongated member (102) including a grip (130) and a scope connector (110) extending proximally therefrom, the grip (130) being sized and shaped for single-handed use.

9. The system of claim 8, further comprising a tenaculum stabilizer (116) located on a proximal portion of the introducer (100).

10. The system of claim 8, wherein a proximal end of the second lumen (128) is in fluid communication with the fluid handling unit (132) to withdraw fluid from the uterus and return fluid withdrawn from the uterus to one of (a) the first lumen (126) for resupply to the uterus and (b) a waste collection unit.

11. The system of claim 7, wherein the elongated member (102) includes a plurality of grooves molded into an outer surface thereof, each of the grooves being positioned proximally adjacent to a corresponding one of the flexible members (122) and sized to receive the corresponding one of the flexible members (122) when compressed thereagainst during insertion through the cervix.

## Patentansprüche

1. Thermische Ablationsvorrichtung, welche aufweist:
ein längliches Teil (102), das bemessen und geformt ist für die Einführung in eine Gebärmutter über eine zervikale Öffnung, wobei das längliche Teil (102) ein erstes Lumen (126) definiert, das sich durch dieses zu einer ersten distalen Öffnung hin erstreckt, die, wenn die Vorrichtung in einer Arbeitsposition ist, konfiguriert ist, sich in die Gebärmutter zu öffnen, und die konfiguriert ist, in einem proximalen Bereich mit einer Quelle für erwärmtes Fluid verbindbar zu sein; und eine Dichtung (120), die auf dem länglichen Teil (102) so positioniert ist, dass, wenn das längliche Teil in der Arbeitsposition ist, die Dichtung (120) in Eingriff mit zervikalem Gewebe um einen Umfang des länglichen Teils herum ist und verhindert, dass in die Gebärmutter über das erste Lumen (126) eingeführte Fluide an dieser vorbei in die Zervix fließen, wobei die Dichtung (120) mehrere flexible Teile (122) enthält, die entlang einer Länge des länglichen Teils (102) voneinander getrennt sind und von der äußeren Oberfläche des länglichen Teils (102) radial nach außen vorstehen, wobei die flexiblen Teile (122) im Wesentlichen scheibenförmig sind und proximal und distal entlang des länglichen Teils (102) ablenkbar sind, wenn sie durch Kontakt mit umgebendem Gewebe zusammengedrückt werden, und wobei ein Abstand zwischen den flexiblen Teilen (122) verhindert, dass ein flexibles Teil (122) ein benachbartes flexibles Teil (122) überlappt, wobei die Vorrichtung weiterhin ein zweites Lumen (128) aufweist, das sich proximal durch das längliche Teil (102) von einer zweiten distalen Öffnung weg erstreckt, die, wenn die Vorrichtung in der Arbeitsposition ist, konfiguriert ist, sich in die Gebärmutter zu öffnen, wobei das zweite Lumen (128) wirksam ist, Fluid aus der Gebärmutter herauszuziehen.

2. Vorrichtung nach Anspruch 1, bei der ein Durchmesser der flexiblen Teile (122) sich entlang einer Länge der Dichtung (120) ändert, wobei die flexiblen Teile (122) einer Verjüngung von einem ersten Punkt zu einem zweiten Punkt entlang einer Länge der Dichtung (120) folgen.

3. Vorrichtung nach Anspruch 1, bei der die Dichtung (120) gebildet ist aus einem von einem länglichen zylindrischen Silikonteil (118), das mit dem länglichen Teil (102) verbunden ist, und einem länglichen zylindrischen Silikonteil (118), das in das längliche Teil (102) geformt ist, wobei die Dichtung (120) weiterhin an einem proximalen Ende von dieser einen Flansch mit vergrößertem Durchmesser aufweist, wobei der Flansch mit vergrößertem Durchmesser so bemessen und geformt ist, dass er mit einer Reibpassung über dem länglichen Teil (102) gleitet.

4. Vorrichtung nach Anspruch 3, weiterhin aufweisend Nuten (216), die in das längliche zylindrische Teil (218) angrenzend an die Dichtung (120) geformt sind, wobei jede der Nuten (216) bemessen ist zur Aufnahme eines entsprechenden der flexiblen Teile (222), wenn diese während der Einführung durch die Zervix gegen diese gedrückt werden.

5. Vorrichtung nach Anspruch 1, bei der jedes der flexiblen Teile (122) eine Dicke von angenähert 0,2 mm und einen äußeren Durchmesser von angenähert 11 mm hat.

6. Vorrichtung nach Anspruch 1, weiterhin aufweisend eine flexible Klappe (630), die von einer äußeren Oberfläche des länglichen Teils (102) distal von den flexiblen Teilen über einen Abstand radial nach außen vorsteht, der größer als ein radialer Abstand der vorstehenden flexiblen Teile (622) ist, wobei ein Durchmesser der Klappe (630) so ausgewählt ist, dass er größer als ein Durchmesser einer Öffnung des zervikalen Kanals in die Gebärmutter ist.

7. Thermisches Ablationssystem, aufweisend die Vorrichtung nach Anspruch 1, wobei das thermische Ablationssystem weiterhin eine Fluidhandhabungseinheit aufweist, die fluidmäßig mit einem proximalen Ende des ersten Lumens (126) gekoppelt ist, welche Fluidhandhabungseinheit (134) erwärmtes Fluid zu dem ersten Lumen (126) liefert, wobei die Dichtung (120) aus Silikon besteht und auf einem distalen Bereich des länglichen Teils (102) positioniert ist.

8. System nach Anspruch 7, weiterhin aufweisend eine Einführungsvorrichtung (100), die mit einem proximalen Ende des länglichen Teils (102) gekoppelt ist, enthaltend einen Griff (130) und einen Scope-Verbinder (110), der sich proximal von diesem weg erstreckt, wobei der Griff (130) für einhändigen Gebrauch bemessen und geformt ist.

9. System nach Anspruch 8, weiterhin aufweisend einen Tenakelstabilisierer (116), der sich auf einem proximalen Bereich der Einführungsvorrichtung (100) befindet.

10. System nach Anspruch 8, bei dem ein proximales Ende des zweiten Lumens (128) in Fluidverbindung mit der Fluidhandhabungseinheit (132) ist, um Fluid aus der Gebärmutter herauszuziehen und aus der Gebärmutter herausgezogenes Fluid zu (a) dem ersten Lumen (126) für die Wiederzuführung zu der Gebärmutter oder (b) einer Abfallsammeleinheit zurückzuführen.

11. System nach Anspruch 7, bei dem das längliche Teil (102) mehrere Nuten enthält, die in eine äußere Oberfläche von diesem geformt sind, wobei jede der Nuten proximal angrenzend an ein entsprechendes der flexiblen Teile (122) positioniert und bemessen ist, das entsprechende der flexiblen Teile (122) aufzunehmen, wenn dieses während der Einführung durch die Zervix gegen diese gedrückt wird.

## Revendications

1. Dispositif d'ablation thermique comprenant :
un élément allongé (102) dimensionné et conformé de manière à pouvoir être inséré à l'intérieur d'un utérus via une ouverture du col de l'utérus, l'élément allongé (102) définissant une première lumière (126) qui s'étend au travers jusqu'à une première ouverture distale qui, lorsque le dispositif est dans une position de fonctionnement, est configurée de manière à déboucher à l'intérieur de l'utérus et qui est configurée de manière à pouvoir être connectée au niveau d'une partie proximale à une source de fluide chauffé ; et un joint (120) positionné sur l'élément allongé (102) de telle sorte que, lorsque l'élément allongé est dans la position de fonctionnement, le joint (120) engage de tissu du col de l'utérus sur une circonférence de l'élément allongé et empêche que des fluides qui sont introduits à l'intérieur de l'utérus via la première lumière (126) ne s'écoulent au-delà à l'intérieur du col de l'utérus, le joint (120) incluant une pluralité d'éléments flexibles (122) séparés les uns des autres suivant une longueur de l'élément allongé (102) et faisant saillie radialement vers l'extérieur depuis une surface externe de l'élément allongé (102), dans lequel les éléments flexibles (122) sont sensiblement en forme de disque et peuvent être déviés de façon proximale et de façon distale suivant l'élément allongé (102) lorsqu'ils sont comprimés par contact avec un tissu avoisinant et dans lequel un espacement entre les éléments flexibles (122) empêche qu'un élément flexible (122) ne chevauche un élément flexible adjacent (122), dans lequel le dispositif comprend en outre une seconde lumière (128) qui s'étend de façon proximale au travers de l'élément allongé (102) depuis une seconde ouverture distale qui, lorsque l'appareil est dans la position de fonctionnement, est configurée de manière à déboucher à l'intérieur de l'utérus, la seconde lumière (128) fonctionnant pour retirer du fluide depuis l'utérus.

2. Dispositif selon la revendication 1, dans lequel un diamètre des éléments flexibles (122) varie suivant une longueur du joint (120), les éléments flexibles (122) décrivant un cône décroissant depuis un premier point jusqu'à un second point suivant une longueur du joint (120).

3. Dispositif selon la revendication 1, dans lequel le joint (120) est formé par un embout pris parmi un embout cylindrique allongé en silicone (118) lié à l'élément allongé (102) et un embout cylindrique allongé en silicone (118) moulé à l'intérieur de l'élément allongé (102), le joint (120) comprenant en outre un embout de diamètre augmenté sur son extrémité proximale, l'embout de diamètre augmenté étant dimensionné et conformé de manière à coulisser au dessus de l'élément allongé (102) selon un ajustement par friction.

4. Dispositif selon la revendication 3, comprenant en outre des gorges (216) moulées à l'intérieur de l'embout cylindrique allongé (218) adjacentes au joint (120), chacune des gorges (216) étant dimensionnée de manière à recevoir l'un correspondant des éléments flexibles (222) lorsqu'il est comprimé contre celle-ci pendant son insertion au travers du col de l'utérus.

5. Dispositif selon la revendication 1, dans lequel chacun des éléments flexibles (122) présente une épaisseur d'approximativement 0,2 mm et un diamètre externe d'approximativement 11 mm.

6. Dispositif selon la revendication 1, comprenant en outre un volet flexible (630) qui est en projection radiale vers l'extérieur depuis une surface externe de l'élément allongé (102) de façon distale par rapport aux éléments flexibles sur une distance supérieure à une distance de projection radiale des éléments flexibles (622), un diamètre du volet (630) étant choisi de manière à être supérieur à un diamètre d'une ouverture du canal du col de l'utérus à l'intérieur de l'utérus.

7. Système d'ablation thermique comprenant le dispositif selon la revendication 1, le système d'ablation thermique comprenant en outre une unité de manipulation de fluide couplée en terme de fluide à une extrémité proximale de la première lumière (126), l'unité de manipulation de fluide (134) alimentant un fluide chauffé jusqu'à la première lumière (126), dans lequel le joint (120) est réalisé en silicone et est positionné sur une partie distale de l'élément allongé (102).

8. Système selon la revendication 7, comprenant en outre un moyen d'introduction (100) couplé à une extrémité proximale de l'élément allongé (102) incluant un moyen de préhension (130) et un connecteur de dispositif de visualisation (110) qui s'étend de façon proximale depuis, le moyen de préhension (130) étant dimensionné et conformé pour une utilisation à une seule main.

9. Système selon la revendication 8, comprenant en outre un stabilisateur de ténaculum (116) situé sur une partie proximale du moyen d'introduction (100).

10. Système selon la revendication 8, dans lequel une extrémité proximale de la seconde lumière (128) est en communication de fluide avec l'unité de manipulation de fluide (132) afin de retirer du fluide depuis l'utérus et de retourner le fluide retiré depuis l'utérus jusqu'à un élément pris parmi (a) la première lumière (126) pour réalimentation sur l'utérus et (b) une unité de collecte de liquide à rejeter.

11. Système selon la revendication 7, dans lequel l'élément allongé (102) inclut une pluralité de gorges moulées à l'intérieur de sa surface externe, chacune des gorges étant positionnée de façon proximale de manière à être adjacente à l'un correspondant des éléments flexibles (122) et étant dimensionnée de manière à recevoir l'un correspondant des éléments flexibles (122) lorsqu'il est comprimé contre celle-ci pendant son insertion au travers du col de l'utérus.
